# EUROPEAN PATENT APPLICATION

(11) **EP 2 374 451 A2**
(43) Date of publication of application: **12.10.2011**
(21) Application number: 11169183.8
(22) Date of filing: 27.07.2006
(51) Int. Cl.: A61K 31/047, A61K 31/11, A61K 31/16, A61K 31/19, A61K 31/223, A61K 31/365, A61K 31/395, A61K 31/4015, A61K 31/473, A61K 31/52, A61K 33/02, A61K 31/00, A61K 45/06, A61P 27/02

(54) **Histone deacetylase inhibitors (HDAC) that correct protein misfolding and uses thereof**

(30) Priority: 27.07.2005 US 703068 P
(62) Divisional of application: 06800453.0
(71) Applicant: University of Florida Research Foundation, Inc., Gainesville, FL 32611-5500 (US)
(72) Inventor: Kaushal, Shalesh, Gainesville, FL Florida 32653 (US); Noorwez, Syed Mohammed, Gainesville, FL Florida 32607 (US)
(74) Representative: Grund, Martin

(57) **Abstract**

The invention features compositions and methods that are useful for treating or preventing a protein conformation disease in a subject by correcting misfolded proteins in vivo. In addition, the invention provides compositions and methods that are useful for expressing a recombinant protein in a biochemically functional conformation.

## Description

### CROSS-REFERENCE TO RELATED APPLICATION

This application claims the benefit of the following U.S. Provisional Application No: 60/703,068, which was filed on July 27, 2005, the contents of which are incorporated herein by reference.

### STATEMENT OF RIGHTS TO INVENTIONS MADE UNDER FEDERALLY

### SPONSORED RESEARCH

This work was supported by a National Eye Institute Grant, Grant No. EY016070-01. The government may have certain rights in the invention.

### BACKGROUND OF THE INVENTION

Proteins must fold into their correct three-dimensional conformation to achieve their biological function. The native conformation of a polypeptide is encoded within its primary amino acid sequence, and even a single mutation in an amino acid sequence can impair the ability of a protein to achieve its proper conformation. When proteins fail to fold correctly, the biological and clinical effects can be devastating. Protein aggregation and misfolding are primary contributors to many human diseases, such as autosomal dominant retinitis pigmentosa, Alzheimer's disease, α1-antitrypsin deficiency, cystic fibrosis, nephrogenic diabetes insipidus, and prion-mediated infections. Disease can result from deficiencies in the level of a protein whose function is required in a particular biochemical pathway, as in cystic fibrosis, where mutations in the cystic fibrosis transmembrane conductance regulator (CFTR), a cAMP-activated chloride channel expressed at the apical membrane of epithelial cells, affect its ability to be made, processed, and trafficked to the plasma membrane, where its function is required. In other protein-folding disorders, disease results because of the cytotoxic effects of the misfolded protein, as in Alzheimer's disease where the aggregation of amyloid plaques cause neuronal damage.

### SUMMARY OF THE INVENTION

The invention features compositions and methods that are useful for treating or preventing a Protein Conformation Disease by correcting misfolded proteins *in vivo* and methods for enhancing the expression of a recombinant proteins in a eukaryotic cell, where the recombinant protein is expressed in a biochemically functional conformation.

In one aspect, the invention generally features methods for treating a subject (e.g., mammal, such as a human) having a protein conformation disorder (PCD) (e.g., α1-antitrypsin deficiency, cystic fibrosis, Huntington's disease, Parkinson's disease, Alzheimer's disease, nephrogenic diabetes insipidus, cancer, and Jacob-Creutzfeld disease). The method involves administering any one or more of the following compounds: a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor, and a histone deacetylase inhibitor, where the compound is administered in an amount sufficient to treat the subject. In one embodiment, the PCD is an ocular PCD selected from the group consisting of retinitis pigmentosa, age-related macular degeneration, glaucoma, corneal dystrophies, retinoschises, Stargardt's disease, autosomal dominant druzen, and Best's macular dystrophy. In another embodiment, the method further involves administering 11-cis-retinal, 9-cis-retinal, or a 7-ring locked isomer of 11-cis-retinal to the subject. The combination of at least one of these compounds and 11-cis-retinal, 9-cis-retinal, or a 7-ring locked isomer of 11-cis-retinal is particularly useful for the treatment of retinitis pigmentosa and age-related macular degeneration.

In other embodiments, where the PCD is cystic fibrosis, the method further involves administering an agent selected from the group consisting of antibiotics, vitamins A, D, E, and K supplements, albuterol bronchodilation, dornase, and ibuprofen; where the PCD is Huntington's disease, the method further involves administering an agent selected from the group consisting of haloperidol, phenothiazine, reserpine, tetrabenazine, amantadine, and co-Enzyme Q10; where the PCD is Parkinson's disease the method further involves administering an agent selected from the group consisting of levodopa, amantadine, bromocriptine, pergolide, apomorphine, benserazide, lysuride, mesulergine, lisuride, lergotrile, memantine, metergoline, piribedil, tyramine, tyrosine, phenylalanine, bromocriptine mesylate, pergolide mesylate, antihistamines, antidepressants, and monoamine oxidase inhibitors; where the PCD is Alzheimer's disease, the method further involves administering an agent selected from the group consisting of donepezil, rivastigmine, galantamine, and tacrine; where the PCD is nephrogenic diabetes insipidus the method further involves administering an agent selected from the group consisting of chlorothiazide/hydrochlorothiazide, amiloride, and indomethacin; and where the PCD is cancer, the method further involves administering an agent selected from the group consisting abiraterone acetate, altretamine, anhydrovinblastine, auristatin, bexarotene, bicalutamide, BMS184476,2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-proly-1-Lproline-t-butylamide, cachectin, cemadotin, chlorambucil, cyclophosphamide, 3',4'-didehydro-4'-deoxy-8'-norvin- caleukoblastine, docetaxol, doxetaxel, carboplatin, carmustine (BCNU),cisplatin, cryptophycin, cyclophosphamide, cytarabine, dacarbazine (DTIC), dactinomycin, daunorubicin, dolastatin, doxorubicin (adriamycin), etoposide, 5-fluorouracil, finasteride, flutamide, hydroxyurea and hydroxyureataxanes, ifosfamide, liarozole, lonidamine, lomustine (CCNU), mechlorethamine (nitrogen mustard), melphalan, mivobulin isethionate, rhizoxin, sertenef, streptozocin, mitomycin, methotrexate, nilutamide, onapristone, paclitaxel, prednimustine, procarbazine, RPR109881, stramustine phosphate, tamoxifen, tasonermin, taxol, tretinoin, vinblastine, vincristine, vindesine sulfate, and vinflunin

In a related aspect, the invention features a method for treating a subject diagnosed as having retinitis pigmentosa. The method involves administering to the subject 11-cis-retinal or 9-cis-retinal; and administering at least one additional compound selected from the group consisting of: a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor, and a histone deacetylase inhibitor, where the 11-cis-retinal or 9-cis-retinal and the compound are administered simultaneously or within fourteen days of each other in amounts sufficient to treat the subject.

In various embodiments of the above aspects, the 11-cis-retinal or 9-cis-retinal and the compound are administered within twenty-four hours of each other, within five, ten or fourteen days of each other; or are administered simultaneously. In other embodiments of the above-aspects, the 11-cis-retinal or 9-cis-retinal and the compound are administered to the eye (e.g., intra-ocularly). In other embodiments of the above-aspects, the 11-cis-retinal or 9-cis-retinal and the compound are each incorporated into a composition that provides for their long-term release (e.g., a microsphere, nanosphere, or nanoemulsion) or their long-term release is achieved using a drug delivery device. In other embodiments of the above-aspects the method further comprises administering a vitamin A supplement.

In yet another aspect, the invention features a method of increasing the amount of a biochemically functional conformation of a protein in a cell. The method involves contacting a cell with an effective amount of at least one compound selected from the group consisting of: a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor, and a histone deacetylase inhibitor; and identifying an increase in the amount of a biochemically functional conformation of the protein. In one embodiment, the method further involves contacting the cell with 11-cis-retinal, 9-cis-retinal, or a 7-ring locked isomer of 11-cis-retinal. In another embodiment, the cell (e.g., a mammalian or human cell in *vitro* or in *vivo*) comprises a mutant protein (e.g., opsin, myocilin, lipofuscin, ßigH3) that forms an aggregate or a fibril protein.

In yet another aspect, the invention features a pharmaceutical composition for the treatment of an ocular PCD comprising an effective amount of 11-cis-retinal or 9-cis-retinal and an effective amount of at least one additional compound selected from the group consisting a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor and a histone deacetylase inhibitor in a pharmaceutically acceptable excipient.

In yet another aspect, the invention features a pharmaceutical composition for the treatment of retinitis pigmentosa comprising an effective amount of 11-cis-retinal or 9-cis-retinal and an effective amount of at least one additional compound selected from the group consisting a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor and a histone deacetylase inhibitor in a pharmaceutically acceptable excipient.

In another aspect, the invention features a kit for the treatment of an ocular PCD. The kit includes an effective amount of 11-cis-retinal or 9-cis-retinal and an effective amount of at least one additional compound selected from the group consisting a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor and a histone deacetylase inhibitor.

In yet another aspect, the invention features a kit for the treatment of retinitis pigmentosa. The kit includes an effective amount of 11-cis-retinal or 9-cis-retinal and an effective amount of at least one additional compound selected from the group consisting a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor and a histone deacetylase inhibitor.

In yet another aspect, the invention features a method for identifying a compound useful for treating a subject having an ocular PCD. The method involves contacting a cell *in vitro* expressing a misfolded protein with a candidate compound; and determining the yield of correctly folded protein recovered from the cell relative to a control cell, where an increase in the yield of correctly folded protein in the contacted cell identifies a compound useful for treating a subject having a PCD.

In yet another aspect, the invention features a method for identifying a compound useful for treating a subject having retinitis pigmentosa. The method involves contacting a cell expressing a misfolded protein *in vitro* with (i) 11-cis-retinal or 9-cis-retinal, and (ii) a candidate compound; and determining the yield of correctly folded protein recovered from the cell relative to a control cell, where an increase in the yield of correctly folded protein in the contacted cell identifies a compound useful for treating a subject having retinitis pigmentosa. In one embodiment, the misfolded protein (e.g., opsin) comprises a mutation (such as a P23H mutation).

In yet another aspect, the invention features a method for treating a subject having a protein conformation disorder (PCD). The method involves administering to the subject a proteasomal inhibitor or an autophagy inhibitor in amounts sufficient to treat the subject.

In yet another aspect, the invention features a method for treating a subject having retinitis pigmentosa. The method involves administering to the subject a proteasomal inhibitor or an autophagy inhibitor (e.g., 3-methyladenine) in amounts sufficient to treat the subject. In one embodiment, the proteasomal inhibitor is a reversible inhibitor of the proteasome (e.g., MG132). In another embodiment, the autophagy inhibitor is selected from the group consisting of 3-methyladenine, 3-methyl adenosine, adenosine, okadaic acid, *N*⁶-mercaptopurine riboside (*N*⁶-MPR), an aminothiolated adenosine analogue, 5-amino-4-imidazole carboxamide riboside (AICAR), and bafilomycin A1.

In yet another aspect, the invention features a method for producing a recombinant protein in a biochemically functional conformation. The method involves contacting a cell (e.g., eukaryotic cell, a yeast cell, a mammalian cell) expressing the recombinant protein with a compound selected from the group consisting of a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor and a histone deacetylase inhibitor; and isolating the recombinant protein from the cell, where the method produces a recombinant protein in a biochemically functional conformation. In one embodiment, the method further involves measuring the biological activity of the protein. In another embodiment, the biological activity is detected using an enzymatic assay or spectrophotometrically. In another embodiment, the method further involves contacting the cell with 11-cis-retinal (e.g., a 7-ring locked isomer of 11-cis-retinal).

In various embodiments of any of the above aspects, the proteasomal inhibitor (e.g., a reversible inhibitor of the proteasome) is any one or more of MG132, lactocystin, clasto-lactocystin-beta-lactone, PSI, MG-115, MG-101, N-Acetyl-Leu-Leu-Met-CHO, N-carbobenzoyl-Gly-Pro-Phe-Leu-CHO, N-carbobenzoyl-Gly-Pro-Ala-Phe-CHO, N-carbobenzoyl-Leu-Leu-Phe-CHO, and salts or analogs thereof; the autophagy inhibitor is any one or more of 3-methyladenine, 3-methyl adenosine, adenosine, okadaic acid, *N*⁶-mercaptopurine riboside (*N*⁶-MPR), 5-amino-4-imidazole carboxamide riboside (AICAR), bafilomycin A1, and salts or analogs thereof; the lysosomal inhibitor is any one or more of leupeptin, trans-epoxysaccinyl-L-leucylamide-(4-guanidino) butane, L-methionine methyl ester, ammonium chloride, methylamine, chloroquine, and salts or analogs thereof; the inhibitor of protein transport from the ER to the Golgi is brefeldin A and salts or analogs thereof; the Hsp90 chaperone inhibitor is any one or more of benzoquinone ansamycin antibiotics, Geldanamycin, 17-allylamino-17-demethoxygeldanamycin, radicicol, novobiocin, and an Hsp90 inhibitor that binds to the Hsp90 ATP/ADP pocket, and salts or analogs thereof; the heat shock response activator is selected from the group consisting of celastrol, celastrol methyl ester, dihydrocelastrol diacetate, celastrol butyl ester, and dihydrocelastrol; the glycosidase inhibitor is any one or more of australine hydrochloride, castanospermine, 6-Acetamido-6-deoxy-castanospermine, deoxyfuconojirimycin hydrochloride (DFJ), deoxynojirimycin (DNJ), deoxygalactonojirimycin hydrochloride (DGJ), deoxymannojirimycin hydrochloride (DMJ), 2R,5R-Bis(hydroxymethyl)-3R,4R-dihydroxypyrrolidine (DMDP), 1,4-Dideoxy-1,4-imino-D-mannitol hydrochloride, 3R,4R,5R,6R)-3,4,5,6-Tetrahydroxyazepane Hydrochloride, 1,5-Dideoxy-1,5-imino-xylitol, Kifumensine, N-butyldeoxynojirimycin (BDNJ), N-nonyl DNJ (NDNJ), N-hexyl DNJ (HDNJ), N-methyldeoxynojirimycin (MDNJ), and salts or analogs thereof; the histone deacetylase inhibitor is any one or more of Scriptaid, APHA Compound 8, Apicidin, sodium butyrate, (-)-Depudecin, Sirtinol, trichostatin A, and salts or analogs thereof. In various embodiments of any of the above aspects, the ocular PCD is any one of age-related macular degeneration, retinitis pigmentosa, glaucoma, coreal systrophy, retinoschises, Stargardt's disease, autosomal dominant druzen, or Best's macular dystrophy, or any other ocular disease characterized by the deposition of protein aggregates or fibrils within a cell of the eye. In various aspects, the mutant protein forming these aggregates or fibrils is opsin, myocilin, lipofuscin, or *BIGH3*βigH3. In other embodiments of any of the above aspects, the subject comprises a mutation that affects protein folding (e.g., a mutation in an opsin, such as a P23H mutation).

### BRIEF DESCRIPTION OF THE DRAWINGS

Figures 1A and 1B are absorbance spectra showing the effect of MG132 and 11-cis-retinal on the absorbance of mutant P23H and wild-type rhodopsin, respectively.
Figures 2A and 2B are absorbance spectra showing the effect of 3-methyladenine and 11-cis-retinal on the absorbance of mutant P23H and wild-type rhodopsin.
Figures 3A and 3B are absorbance spectra showing the effect of ammonium chloride and 11-cis-retinal on the absorbance of mutant P23H and wild-type rhodopsin.
Figures 4A and 4B are absorbance spectra showing the effect of brefeldin A and 11-cis-retinal on the absorbance of mutant P23H and wild-type rhodopsin.
Figures 5A and 5B are absorbance spectra showing the effect of Geldanamycin and 11-cis-retinal on the absorbance of mutant P23H and wild-type rhodopsin.
Figures 6A and 6B are absorbance spectra showing the effect of Celastrol and 11-cis-retinal on the absorbance of mutant P23H and wild-type rhodopsin.
Figures 7A and 7B are absorbance spectra showing the effect of Dihydro-celastrol and 11-cis-retinal on the absorbance of mutant P23H and wild-type rhodopsin.
Figures 8A and 8B are absorbance spectra showing the effect of Scriptaid and 11-cis-retinal on the absorbance of mutant P23H and wild-type rhodopsin.

### DETAILED DESCRIPTION OF THE INVENTION

### Definitions

By "protein conformational disease" is meant a disease or disorder whose pathology is related to the presence of a misfolded protein. In one embodiment, a protein conformational disease is caused when a misfolded protein interferes with the normal biological activity of a cell, tissue, or organ.

By "proteasomal inhibitor" is meant a compound that reduces a proteasomal activity, such as the degradation of a ubiquinated protein.

By "autophagy inhibitor" is meant a compound that reduces the degradation of a cellular component by a cell in which the component resides.

By "lysosomal inhibitor" is meant a compound that reduces the intracellular digestion of macromolecules by a lysosome. In one embodiment, a lysosomal inhibitor decreases the proteolytic activity of a lysosome.

By "inhibitor of ER-Golgi protein transport" is meant a compound that reduces the transport of a protein from the ER to the Golgi, or from the Golgi to the ER.

By "Hsp90 chaperone inhibitor" is meant a compound that reduces the chaperone activity of Hsp90. In one embodiment, the inhibitor alters protein binding to an Hsp90 ATP/ADP pocket.

By "heat shock response activator" is meant a compound that increases the chaperone activity or expression of a heat shock pathway component. Heat shock pathway components include, but are not limited to, Hsp100, Hsp90, Hsp70, Hsp60, Hsp40 and small HSP family members.

By "glycosidase inhibitor" is meant a compound that reduces the activity of an enzyme that cleaves a glycosidic bond.

By "histone deacetylase inhibitor" is meant a compound that reduces the activity of an enzyme that deacetylates a histone.

By "reduces" or "increases" is meant a negative or positive alteration, respectively, of at least 10%, 25%, 50%, 75%, or 100%

By "a biochemically functional conformation" is meant that a protein has a tertiary structure that permits the protein to be biologically active. When a mutant protein assumes a biochemically functional conformation its biological activity is increased. Accordingly, a mutant protein having a biochemically functional conformation may, to some degree, functionally substitute for a wild-type protein.

### Methods of the Invention

In one embodiment, the present invention provides methods of treating disease and/or disorders or symptoms thereof which comprise administering a therapeutically effective amount of a pharmaceutical composition comprising a compound of the formulae herein to a subject (e.g., a mammal such as a human). Thus, one embodiment is a method of treating a subject suffering from or susceptible to a protein conformation disease or disorder or symptom thereof. The method includes the step of administering to the mammal a therapeutic amount of an amount of a compound herein sufficient to treat the disease or disorder or symptom thereof, under conditions such that the disease or disorder is treated.

The methods herein include administering to the subject (including a subject identified as in need of such treatment) an effective amount of a compound described herein, or a composition described herein to produce such effect. Identifying a subject in need of such treatment can be in the judgment of a subject or a health care professional and can be subjective (e.g. opinion) or objective (e.g. measurable by a test or diagnostic method).

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

As used herein, the terms "prevent," "preventing," "prevention," "prophylactic treatment" and the like refer to reducing the probability of developing a disorder or condition in a subject, who does not have, but is at risk of or susceptible to developing a disorder or condition.

The therapeutic methods of the invention (which include prophylactic treatment) in general comprise administration of a therapeutically effective amount of the compounds herein, such as a compound of the formulae herein to a subject (e.g., animal, human) in need thereof, including a mammal, particularly a human. Such treatment will be suitably administered to subjects, particularly humans, suffering from, having, susceptible to, or at risk for a disease, disorder, or symptom thereof. Determination of those subjects "at risk" can be made by any objective or subjective determination by a diagnostic test or opinion of a subject or health care provider (e.g., genetic test, enzyme or protein marker, Marker (as defined herein), family history, and the like). The compounds herein may be also used in the treatment of any other disorders in which protein folding (including misfolding) may be implicated.

The invention features compositions and methods that are useful for correcting misfolded proteins *in vivo.* Misfolded proteins can interfere with normal cell function, and can result in a human Protein Conformational Disease (PCD). PCDs include α1-antitrypsin deficiency, cystic fibrosis, Huntington's disease, Parkinson's disease, Alzheimer's disease, nephrogenic diabetes insipidus, cancer, and prion-related disorders (e.g., Jacob-Creutzfeld disease). The compositions and methods of the invention are particularly useful for the prevention or treatment of ocular PCDs, including retinitis pigmentosa, age-related macular degeneration, glaucoma, corneal dystrophies, retinoschises, Stargardt's disease, autosomal dominant druzen, Best's macular dystrophy, and corneal dystrophies. Compositions of the invention can be used to treat the PCD, to slow the death of affected cells, to relieve symptoms caused by the PCD, or to prevent a PCD from being initiated in the first place.

The invention is generally based on the discovery that 11-cis-retinal in combination with a proteasomal inhibitor, an autophagy inhibitor, a lysosomal inhibitor, an inhibitor of protein transport from the ER to the Golgi, an Hsp90 chaperone inhibitor, a heat shock response activator, or a histone deacetylase inhibitor can be used to correct the conformation of a misfolded opsin protein or to increase the amount of correctly folded protein in a cell. Specifically, 11-cis-retinal in combination with the proteasomal inhibitor MG132, the autophagy inhibitor 3-methyladenine, the lysosomal inhibitor ammonium chloride, the ER-Golgi transport inhibitor brefeldin A, the Hsp90 inhibitor Geldanamycin, the heat shock response activator Celastrol, or the histone deacetylase inhibitor Scriptaid allowed a mutant P23H opsin protein to assume a biochemically functional conformation and associate with 11-cis-retinal to form rhodopsin. In addition, the proteasomal inhibitor MG132 and the autophagic inhibitor 3-methyl adenine were each used independently to correct the conformation of mutant P23H opsin and allow it to form rhodopsin.

### Proteasomal Inhibitors

The 26S proteasome is a multicatalytic protease that cleaves ubiquinated proteins into short peptides. Proteasomal inhibitors are one class of compounds that can be used independently or in combination with 11-cis-retinal, 9-cis-retinal, or a 7-ring locked isomer of 11-*cis*-retinal for the treatment of PCD. MG-132 is one proteasomal inhibitor that may be used independently or in combination with 11-cis-retinal, 9-cis-retinal, or a 7-ring locked isomer of 11-*cis*-retinal. MG-132 is particularly useful for the treatment of retinitis pigmentosa and other ocular diseases related to protein aggregation or protein misfolding. Other proteasomal inhibitors useful in the methods of the invention include lactocystin (LC), clasto-lactocystin-beta-lactone, PSI (N-carbobenzoyl-Ile-Glu-(OtBu)-Ala-Leu-CHO), MG-132 (N-carbobenzoyl-Leu-Leu-Leu-CHO).MG-115(N-carbobenzoyl-Leu-Leu-Nva-CHO), MG-101 (N-Acetyl-Leu-Leu-norLeu-CHO), ALLM (N-Acetyl-Leu-Leu-Met-CHO), N-carbobenzoyl-Gly-Pro-Phe-Leu-CHO, N-carbobenzoyl-Gly-Pro-Ala-Phe-CHO, N-carbobenzoyl-Leu-Leu-Phe-CHO, and salts or analogs thereof. Other proteasomal inhibitors and their uses are described for example, in U.S. Patent No. 6,492,333.

### Autophagy Inhibitors

Autophagy is an evolutionarily conserved mechanism for the degradation of cellular components in the cytoplasm, and serves as a cell survival mechanism in starving cells. During autophagy pieces of cytoplasm become encapsulated by cellular membranes, forming autophagic vacuoles that eventually fuse with lysosomes to have their contents degraded. Autophagy inhibitors may be used independently or in combination with 11-cis-retinal, 9-cis-retinal, or a 7-ring locked isomer of 11-*cis*-retinal for the treatment of PCD. The autophagy inhibitor 3-methyl adenine is particularly useful for the treatment of retinitis pigmentosa or other ocular diseases related to misfolded proteins or protein aggregation. Autophagy inhibitors useful in the methods of the invention include, but are not limited to, 3-methyladenine, 3-methyl adenosine, adenosine, okadaic acid, *N*⁶-mercaptopurine riboside (*N*⁶-MPR), an aminothiolated adenosine analogue, 5-amino-4-imidazole carboxamide riboside (AICAR), bafilomycin A1, and salts or analogs thereof.

### Lysosomal Inhibitors

The lysosome is a major site of cellular protein degradation. Degradation of proteins entering the cell by receptor-mediated endocytosis or by pinocytosis, and of plasma membrane proteins takes place in lysosomes. Lysosomal inhibitors, such as ammonium chloride, leupeptin, trans-epoxysaccinyl-L-leucylamide-(4-guanidino) butane, L-methionine methyl ester, ammonium chloride, methylamine, chloroquine, and salts or analogs thereof, are useful in combination with 11-cis-retinal, 9-cis-retinal, or a 7-ring locked isomer of 11-*cis-*retinal for the treatment of PCD.

### ER-Golgi Transport Inhibitors

Newly synthesized proteins enter the biosynthetic- secretory pathway in the ER. To exit from the ER, the proteins must be properly folded. Those proteins that are misfolded are retained in the ER ER-Golgi transport inhibitors are useful for the treatment of PCD. Brefeldin A is one exemplary ER-Golgi transport inhibitor that is useful in the methods of the invention.

### HSP90 Chaperone Inhibitors

Heat shock protein 90 (Hsp90) is responsible for chaperoning proteins involved in cell signaling, proliferation and survival, and is essential for the conformational stability and function of a number of proteins. HSP-90 inhibitors are useful in combination with 11-cis-retinal, 9-cis-retinal, or a 7-ring locked isomer of 11-*cis*-retinal for the treatment of PCD. HSP-90 inhibitors include benzoquinone ansamycin antibiotics, such as geldanamycin and 17-allylamino-17-demethoxygeldanamycin (17-AAG), which specifically bind to Hsp90, alter its function, and promote the proteolytic degradation of substrate proteins. Other HSP-90 inhibitors include, but are not limited to, radicicol, novobiocin, and any Hsp90 inhibitor that binds to the Hsp90 ATP/ADP pocket.

### Heat Shock Response Activators

Celastrol, a quinone methide triterpene, activates the human heat shock response. In combination with 11-cis-retinal, 9-cis-retinal, or a 7-ring locked isomer of 11-*cis*-retinal, celastrol and other heat shock response activators are useful for the treatment of PCD. Heat shock response activators include, but are not limited to, celastrol, celastrol methyl ester, dihydrocelastrol diacetate, celastrol butyl ester, dihydrocelastrol, and salts or analogs thereof.

### Histone Deacetylase Inhibitors

Regulation of gene expression is mediated by several mechanisms, including the post-translational modifications of histones by dynamic acetylation and deacetylation. The enzymes responsible for reversible acetylation/-deacetylation processes are histone acetyltransferases (HATs) and histone deacetylases (HDACs), respectively. Histone deacetylase inhibitors include Scriptaid, APHA Compound 8, Apicidin, sodium butyrate, (-)-Depudecin, Sirtinol, trichostatin A, and salts or analogs thereof.

### Glycosidase Inhibitors

Glycosidase inhibitors are one class of compounds that are useful for the treatment of a protein conformation disease, particularly when administered in combination with 11-cis-retinal, 9-cis-retinal, or a 7-ring locked isomer of 11-cis-retinal. Castanospermine, which is a polyhydroxy alkaloid isolated from plant sources, inhibits enzymatic glycoside hydrolysis. Castanospermine and its derivatives are particularly useful for the treatment of a PCD, such as retinitis pigmentosa. Also useful in the methods of the invention are other glycosidase inhibitors, including australine hydrochloride, 6-Acetamido-6-deoxy-castanospermine, which is A powerful inhibitor of hexosaminidases, Deoxyfuconojirimycin hydrochloride (DFJ), Deoxynojirimycin (DNJ), which inhibits glucosidase I and II, Deoxygalactonojirimycin hydrochloride (DGJ), which inhibits α-D-galactosidase, Deoxymannojirimycin hydrochloride (DMJ), 2R,5R-Bis(hydroxymethyl)-3R,4R-dihydroxypyrrolidine (DMDP), also known as 2,5-dideoxy-2,5- imino-D-mannitol, 1,4-Dideoxy-1,4-imino-D-mannitol hydrochloride, 3R,4R,5R,6R)-3,4,5,6-Tetrahydroxyazepane Hydrochloride, which inhibits b-N-acetylglucosaminidase, 1,5-Dideoxy-1,5-imino-xylitol, which inhibits β-glucosidase, and Kifunensine, an inhibitor of mannosidase 1. Also useful in combination with 9-cis-retinal or 11-cis-retinal are N-butyldeoxynojirimycin (BDNJ), N-nonyl DNJ (NDNJ), N-hexyl DNJ (HDNJ), N-methyldeoxynojirimycin (MDNJ), and other glycosidase inhibitors known in the art. Glycosidase inhibitors are available commercially, for example, from Industrial Research Limited (Wellington, New Zealand) and methods of using them are described, for example, in U.S. Patent Nos. 4,894,388, 5,043,273, 5,103,008, 5,844,102, and 6,831,176; and in U.S. Patent Publication Nos. 20020006909.

### Ocular Protein Conformational Disorders

Compositions of the invention are particularly useful for the treatment of virtually any ocular protein conformational disorder (PCD). Such disorders are characterized by the accumulation of misfolded proteins as protein aggragates or fibrils within the eye. Retinitis pigmentosa is an exemplary ocular PCD that is associated with the misfolding of an opsin (e.g., P23H opsin) (GenBank Accession Nos. NM_000539 and NP_000530), as well as with mutations in carbonic anhydrase IV (CA4) )(GenBank Accession Nos. NM_000717 and NP_000708) (Rebello et al., Proc Natl Acad Sci U S A. 2004 Apr 27;101(17):6617-22). CA4 is a glycosylphosphatidylinositol-anchored protein that is highly expressed in the choriocapillaris of the human eye. An R14W mutation causes the CA4 protein to be incorrectly folded and patients carrying this mutation suffer from autosomal dominant retinitis pigmentosa. Compositions of the invention that increase the amount of CA4 in a biochemically functional conformation are useful for the treatment of autosomal dominant retinitis pigmentosa associated with mutations in the CA4 polypeptide.

X-linked juvenile retinoschisis (RS) is another ocular PCD. RS is a common cause of juvenile macular degeneration in males. Mutations in RS1 (NM_000330, NP_000321), or retinoschesin, are responsible for X-linked retinoschisis, a common, early-onset macular degeneration in males that results in a splitting of the inner layers of the retina and severe loss in vision. Mutations in RS 1 disrupt protein folding (J Biol Chem. 2005 Mar 18;280(11):10721-30). Compositions of the invention that increase the amount of RS1 in a biochemically functional conformation are useful for the treatment of retinoschisis.

Glaucoma is an ocular PCD that is associated with mutations in myocilin. Myocilin is a secreted glycoprotein of unknown function that is ubiquitously expressed in many human organs, including the eye. Mutations in this the myocilin protein cause one form of glaucoma, a leading cause of blindness worldwide. Mutant myocilins accumulate in the endoplasmic reticulum of transfected cells as insoluble aggregates (Aroca-Aguilar et al., Biol Chem. 2005 Jun 3;280(22):21043-51; GenBank Accession Nos. NM_000261 and NP_000252). Compositions of the invention that increase the amount of myocilin in a biochemically functional conformation are useful for the treatment of myocilin-associated glaucoma.

Stargardt-like macular degeneration is an ocular PCD that is associated with mutations in ELOVL4. ELOVL4 (Elongation of very long chain fatty acids 4) is a member of the ELO family of proteins involved in the biosynthesis of very long chain fatty acids. Mutations in ELOVL4 have been identified in patients with autosomal dominant Stargardt-like macular degeneration (STGD3/adMD). ELOVL4 mutant proteins accumulate as large aggregates in transfected cells (Grayson et al., J Biol Chem. 2005 Jul 21; Epub) (GenBank Accession Nos. NM_022726 and NP_073563). Compositions of the invention that increase the amount of ELOVL4 in a biochemically functional conformation are useful for the treatment of Stargardt-like macular degeneration.

Malattia Leventinese (ML) and Doyne honeycomb retinal dystrophy (DHRD) refer to two autosomal dominant PCDs that are characterized by yellow-white deposits known as drusen that accumulate beneath the retinal pigment epithelium (RPE). EFEMP1 has a role in retinal drusen formation and is involved in the etiology of macular degeneration (Stone et al., Nat Genet. 1999 Jun;22(2):199-202) (GenBank Accession Nos NM_004105 and NP_004096). Mutant EFEMP1 is misfolded and retained within cells. Compositions of the invention that increase the amount of EFEMP1 in a biochemically functional conformation are useful for the treatment of autosomal dominant drusen.

Best's macular dystrophy is an autosomal dominant PCD that is caused by mutations in VMD2 (hBEST1), which encodes Bestrophin, a Cl(-) channels (Gomez et al., DNA Seq. 2001 Dec;12(5-6):431-5) (GenBank Accession Nos: NM_004183 andNP_004174). Mutations in bestrophin likely cause protein misfolding. Compositions of the invention that increase the amount of correctly folded bestrophin are useful for the treatment of Best's macular dystrophy.

5q31-linked corneal dystrophies are autosomal dominant PCDs that are characterized by age-dependent progressive accumulation ofprotein deposits in the cornea followed by visual impairment. Mutations in the *BIGH3* gene (GenBank Accession No: NM_000358), also termed *TGFBI* (transforming growth factor-β-induced) were found to be responsible for this entire group of conditions. Substitutions at the Arg-124 as well as other residues result in cornea-specific deposition of the encoded protein (GenBank Accession No. NP_000349) via distinct aggregation pathways that involve altered turnover of the protein in corneal tissue. Compositions of the invention that increase the amount of correctly folded TGFBI protein are useful for the treatment of 5q31-linked corneal dystrophies.

In one embodiment, the invention provides a method of monitoring treatment progress. The method includes the step of determining a level of diagnostic marker (Marker) (e.g., any target delineated herein modulated by a compound herein, a protein or indicator thereof, etc.) or diagnostic measurement (e.g., screen, assay) in a subject suffering from or susceptible to a disorder or symptoms thereof associated with protein folding (including misfolding), in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

### Pharmaceutical Compositions

The present invention features pharmaceutical preparations comprising compounds together with pharmaceutically acceptable carriers, where the compounds provide for the generation of a mutant protein in a biochemically functional conformation. Such preparations have both therapeutic and prophylactic applications. In one embodiment, a pharmaceutical composition includes 11-cis-retinal or 9-cis-retinal in combination with at least one additional compound that is a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor, or a histone deacetylase inhibitor. The 11-cis-retinal or 9-cis-retinal and the second compound are formulated together or separately. In another embodiment, a pharmaceutical composition includes a proteasomal inhibitor or an autophagy inhibitor. Compounds of the invention may be administered as part of a pharmaceutical composition. The compositions should be sterile and contain a therapeutically effective amount of the polypeptides in a unit of weight or volume suitable for administration to a subject The compositions and combinations of the invention can be part of a pharmaceutical pack, where each of the compounds is present in individual dosage amounts.

Pharmaceutical compositions of the invention to be used for prophylactic or therapeutic administration should be sterile. Sterility is readily accomplished by filtration through sterile filtration membranes (e.g., 0.2 µm membranes), by gamma irradiation, or any other suitable means known to those skilled in the art. Therapeutic polypeptide compositions generally are placed into a container having a sterile access port, for example, an intravenous solution bag or vial having a stopper pierceable by a hypodermic injection needle. These compositions ordinarily will be stored in unit or multi-dose containers, for example, sealed ampoules or vials, as an aqueous solution or as a lyophilized formulation for reconstitution.

The compounds may be combined, optionally, with a pharmaceutically acceptable excipient. The term "pharmaceutically-acceptable excipient" as used herein means one or more compatible solid or liquid filler, diluents or encapsulating substances that are suitable for administration into a human. The term "carrier" denotes an organic or inorganic ingredient, natural or synthetic, with which the active ingredient is combined to facilitate administration. The components of the pharmaceutical compositions also are capable of being co-mingled with the molecules of the present invention, and with each other, in a manner such that there is no interaction that would substantially impair the desired pharmaceutical efficacy.

Compounds of the present invention can be contained in a pharmaceutically acceptable excipient. The excipient preferably contains minor amounts of additives such as substances that enhance isotonicity and chemical stability. Such materials are non-toxic to recipients at the dosages and concentrations employed, and include buffers such as phosphate, citrate, succinate, acetate, lactate, tartrate, and other organic acids or their salts; tris- hydroxymethylaminomethane (TRIS), bicarbonate, carbonate, and other organic bases and their salts; antioxidants, such as ascorbic acid; low molecular weight (for example, less than about ten residues) polypeptides, e.g., polyarginine, polylysine, polyglutamate and polyaspartate; proteins, such as serum albumin, gelatin, or immunoglobulins; hydrophilic polymers, such as polyvinylpyrrolidone (PVP), polypropylene glycols (PPGs), and polyethylene glycols (PEGs); amino acids, such as glycine, glutamic acid, aspartic acid, histidine, lysine, or arginine; monosaccharides, disaccharides, and other carbohydrates including cellulose or its derivatives, glucose, mannose, sucrose, dextrins or sulfated carbohydrate derivatives, such as heparin, chondroitin sulfate or dextran sulfate; polyvalent metal ions, such as divalent metal ions including calcium ions, magnesium ions and manganese ions; chelating agents, such as ethylenediamine tetraacetic acid (EDTA); sugar alcohols, such as mannitol or sorbitol; counterions, such as sodium or ammonium; and/or nonionic surfactants, such as polysorbates or poloxamers. Other additives may be included, such as stabilizers, anti-microbials, inert gases, fluid and nutrient replenishers (i.e., Ringer's dextrose), electrolyte replenishers, and the like, which can be present in conventional amounts.

The compositions, as described above, can be administered in effective amounts. The effective amount will depend upon the mode of administration, the particular condition being treated and the desired outcome. It may also depend upon the stage of the condition, the age and physical condition of the subject, the nature of concurrent therapy, if any, and like factors well known to the medical practitioner. For therapeutic applications, it is that amount sufficient to achieve a medically desirable result.

With respect to a subject having a protein conformation disease or disorder, an effective amount is sufficient to increase the level of a correctly folded protein in a cell. With respect to a subject having a disease or disorder related to a misfolded protein, an effective amount is an amount sufficient to stabilize, slow, or reduce the a symptom associated with a pathology. Generally, doses of the compounds of the present invention would be from about 0.01 mg/kg per day to about 1000 mg/kg per day. It is expected that doses ranging from about 50 to about 2000 mg/kg will be suitable. Lower doses will result from certain forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day are contemplated to achieve appropriate systemic levels of a composition of the present invention.

A variety of administration routes are available. The methods of the invention, generally speaking, may be practiced using any mode of administration that is medically acceptable, meaning any mode that produces effective levels of the active compounds without causing clinically unacceptable adverse effects. In one preferred embodiment, a composition of the invention is administered intraocularly. Other modes of administration include oral, rectal, topical, intraocular, buccal, intravaginal, intracisternal, intracerebroventricular, intratracheal, nasal, transdermal, within/on implants, or parenteral routes. The term "parenteral" includes subcutaneous, intrathecal, intravenous, intramuscular, intraperitoneal, or infusion. Compositions comprising a composition of the invention can be added to a physiological fluid, such as to the intravitreal humor. For CNS administration, a variety of techniques are available for promoting transfer of the therapeutic across the blood brain barrier including disruption by surgery or injection, drugs which transiently open adhesion contact between the CNS vasculature endothelial cells, and compounds that facilitate translocation through such cells. Oral administration can be preferred for prophylactic treatment because of the convenience to the patient as well as the dosing schedule.

Pharmaceutical compositions of the invention can optionally further contain one or more additional proteins as desired, including plasma proteins, proteases, and other biological material, so long as it does not cause adverse effects upon administration to a subject. Suitable proteins or biological material may be obtained from human or mammalian plasma by any of the purification methods known and available to those skilled in the art; from supernatants, extracts, or lysates of recombinant tissue culture, viruses, yeast, bacteria, or the like that contain a gene that expresses a human or mammalian plasma protein which has been introduced according to standard recombinant DNA techniques; or from the fluids (e.g., blood, milk, lymph, urine or the like) or transgenic animals that contain a gene that expresses a human plasma protein which has been introduced according to standard transgenic techniques.

Pharmaceutical compositions of the invention can comprise one or more pH buffering compounds to maintain the pH of the formulation at a predetermined level that reflects physiological pH, such as in the range of about 5.0 to about 8.0. The pH buffering compound used in the aqueous liquid formulation can be an amino acid or mixture of amino acids, such as histidine or a mixture of amino acids such as histidine and glycine. Alternatively, the pH buffering compound is preferably an agent which maintains the pH of the formulation at a predetermined level, such as in the range of about 5.0 to about 8.0, and which does not chelate calcium ions. Illustrative examples of such pH buffering compounds include, but are not limited to, imidazole and acetate ions. The pH buffering compound may be present in any amount suitable to maintain the pH of the formulation at a predetermined level.

Pharmaceutical compositions of the invention can also contain one or more osmotic modulating agents, i.e., a compound that modulates the osmotic properties (e.g, tonicity, osmolality and/or osmotic pressure) of the formulation to a level that is acceptable to the blood stream and blood cells of recipient individuals. The osmotic modulating agent can be an agent that does not chelate calcium ions. The osmotic modulating agent can be any compound known or available to those skilled in the art that modulates the osmotic properties of the formulation. One skilled in the art may empirically determine the suitability of a given osmotic modulating agent for use in the inventive formulation. Illustrative examples of suitable types of osmotic modulating agents include, but are not limited to: salts, such as sodium chloride and sodium acetate; sugars, such as sucrose, dextrose, and mannitol; amino acids, such as glycine; and mixtures of one or more of these agents and/or types of agents. The osmotic modulating agent(s) may be present in any concentration sufficient to modulate the osmotic properties of the formulation.

Compositions comprising a compound of the present invention can contain multivalent metal ions, such as calcium ions, magnesium ions and/or manganese ions. Any multivalent metal ion that helps stabilizes the composition and that will not adversely affect recipient individuals may be used. The skilled artisan, based on these two criteria, can determine suitable metal ions empirically and suitable sources of such metal ions are known, and include inorganic and organic salts.

Pharmaceutical compositions of the invention can also be a non-aqueous liquid formulation. Any suitable non-aqueous liquid may be employed, provided that it provides stability to the active agents (s) contained therein. Preferably, the non-aqueous liquid is a hydrophilic liquid. Illustrative examples of suitable non-aqueous liquids include: glycerol; dimethyl sulfoxide (DMSO); polydimethylsiloxane (PMS); ethylene glycols, such as ethylene glycol, diethylene glycol, triethylene glycol, polyethylene glycol ("PEG") 200, PEG 300, and PEG 400; and propylene glycols, such as dipropylene glycol, tripropylene glycol, polypropylene glycol ("PPG") 425, PPG 725, PPG 1000, PPG 2000, PPG 3000 and PPG 4000.

Pharmaceutical compositions of the invention can also be a mixed aqueous/non-aqueous liquid formulation. Any suitable non-aqueous liquid formulation, such as those described above, can be employed along with any aqueous liquid formulation, such as those described above, provided that the mixed aqueous/non-aqueous liquid formulation provides stability to the compound contained therein. Preferably, the non- aqueous liquid in such a formulation is a hydrophilic liquid. Illustrative examples of suitable non-aqueous liquids include: glycerol; DMSO; PMS; ethylene glycols, such as PEG 200, PEG 300, and PEG 400; and propylene glycols, such as PPG 425, PPG 725, PPG 1000, PPG 2000, PPG 3000 and PPG 4000.

Suitable stable formulations can permit storage of the active agents in a frozen or an unfrozen liquid state. Stable liquid formulations can be stored at a temperature of at least - 70°C, but can also be stored at higher temperatures of at least 0°C, or between about 0.1°C and about 42°C, depending on the properties of the composition. It is generally known to the skilled artisan that proteins and polypeptides are sensitive to changes in pH, temperature, and a multiplicity of other factors that may affect therapeutic efficacy.

In certain embodiments a desirable route of administration can be by pulmonary aerosol. Techniques for preparing aerosol delivery systems containing polypeptides are well known to those of skill in the art. Generally, such systems should utilize components that will not significantly impair the biological properties of the antibodies, such as the paratope binding capacity (see, for example, Sciarra and Cutie, "Aerosols," in Remington's Pharmaceutical Sciences, 18th edition, 1990, pp 1694-1712; incorporated by reference). Those of skill in the art can readily modify the various parameters and conditions for producing polypeptide aerosols without resorting to undue experimentation.

Other delivery systems can include time-release, delayed release or sustained release delivery systems. Such systems can avoid repeated administrations of compositions of the invention, increasing convenience to the subject and the physician. Many types of release delivery systems are available and known to those of ordinary skill in the art. They include polymer base systems such as polylactides (U.S. Pat. No. 3,773,919; European Patent No. 58,481), poly(lactide-glycolide), copolyoxalates, polycaprolactones, polyesteramides, polyorthoesters, polyhydroxybutyric acids, such as poly-D-(-)-3-hydroxybutyric acid (European Patent No. 133, 988), copolymers of L-glutamic acid and gamma-ethyl-L-glutamate (Sidman, K.R. et al., Biopolymers 22: 547-556), poly (2-hydroxyethyl methacrylate) or ethylene vinyl acetate (Langer, R. et al., J. Biomed. Mater. Res. 15:267-277; Langer, R. Chem. Tech. 12:98-105), and polyanhydrides.

Other examples of sustained-release compositions include semi-permeable polymer matrices in the form of shaped articles, e.g., films, or microcapsules. Delivery systems also include non-polymer systems that are: lipids including sterols such as cholesterol, cholesterol esters and fatty acids or neutral fats such as mono- di- and tri-glycerides; hydrogel release systems such as biologically-derived bioresorbable hydrogel (i.e., chitin hydrogels or chitosan hydrogels); sylastic systems; peptide based systems; wax coatings; compressed tablets using conventional binders and excipients; partially fused implants; and the like. Specific examples include, but are not limited to: (a) erosional systems in which the agent is contained in a form within a matrix such as those described in U.S. Patent Nos. 4,452,775, 4,667,014, 4,748,034 and 5,239,660 and (b) diffusional systems in which an active component permeates at a controlled rate from a polymer such as described in U.S. Patent Nos. 3,832,253, and 3,854,480.

Another type of delivery system that can be used with the methods and compositions of the invention is a colloidal dispersion system. Colloidal dispersion systems include lipid-based systems including oil-in-water emulsions, micelles, mixed micelles, and liposomes. Liposomes are artificial membrane vessels, which are useful as a delivery vector *in vivo* or *in vitro.* Large unilamellar vessels (LUV), which range in size from 0.2 - 4.0 µm, can encapsulate large macromolecules within the aqueous interior and be delivered to cells in a biologically active form (Fraley, R., and Papahadjopoulos, D., Trends Biochem. Sci. 6: 77-80).

Liposomes can be targeted to a particular tissue by coupling the liposome to a specific ligand such as a monoclonal antibody, sugar, glycolipid, or protein. Liposomes are commercially available from Gibco BRL, for example, as LIPOFECTIN™ and LIPOFECTACE™, which are formed of cationic lipids such as N-[1-(2, 3 dioleyloxy)-propyl]-N, N, N-trimethylammonium chloride (DOTMA) and dimethyl dioctadecylammonium bromide (DDAB). Methods for making liposomes are well known in the art and have been described in many publications, for example, in DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Liposomes also have been reviewed by Gregoriadis, G., Trends Biotechnol., 3: 235-241).

Another type of vehicle is a biocompatible microparticle or implant that is suitable for implantation into the mammalian recipient Exemplary bioerodible implants that are useful in accordance with this method are described in PCT International application no. PCT/US/03307 (Publication No. WO 95/24929, entitled "Polymeric Gene Delivery System"). PCT/US/0307 describes biocompatible, preferably biodegradable polymeric matrices for containing an exogenous gene under the control of an appropriate promoter. The polymeric matrices can be used to achieve sustained release of the exogenous gene or gene product in the subject.

The polymeric matrix preferably is in the form of a microparticle such as a microsphere (wherein an agent is dispersed throughout a solid polymeric matrix) or a microcapsule (wherein an agent is stored in the core of a polymeric shell). Microcapsules of the foregoing polymers containing drugs are described in, for example, U.S. Patent 5,075,109. Other forms of the polymeric matrix for containing an agent include films, coatings, gels, implants, and stents. The size and composition of the polymeric matrix device is selected to result in favorable release kinetics in the tissue into which the matrix is introduced. The size of the polymeric matrix further is selected according to the method of delivery that is to be used. Preferably, when an aerosol route is used the polymeric matrix and composition are encompassed in a surfactant vehicle. The polymeric matrix composition can be selected to have both favorable degradation rates and also to be formed of a material, which is a bioadhesive, to further increase the effectiveness of transfer. The matrix composition also can be selected not to degrade, but rather to release by diffusion over an extended period of time. The delivery system can also be a biocompatible microsphere that is suitable for local, site-specific delivery. Such microspheres are disclosed in Chickering, D.E., et al., Biotechnol. Bioeng., 52: 96-101; Mathiowitz, E., et al., Nature 386: 410-414.

Both non-biodegradable and biodegradable polymeric matrices can be used to deliver the compositions of the invention to the subject. Such polymers may be natural or synthetic polymers. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is cross-linked with multivalent ions or other polymers.

Exemplary synthetic polymers which can be used to form the biodegradable delivery system include: polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene, polyvinylpyrrolidone, and polymers of lactic acid and glycolic acid, polyanhydrides, poly(ortho)esters, poly(butic acid), poly(valeric acid), and poly(lactide-cocaprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water *in vivo,* by surface or bulk erosion.

### Methods of Ocular Delivery

The compositions of the invention (e.g., proteasomal inhibitor, an autophagy inhibitor, a lysosomal inhibitor, an inhibitor of protein transport from the ER to the Golgi, an Hsp90 chaperone inhibitor, a heat shock response activator, or a histone deacetylase inhibitor) are particularly suitable for treating ocular protein conformation diseases, such as glaucoma, retinitis pigmentosa, age-related macular degeneration, glaucoma, corneal dystrophies, retinoschises, Stargardt's disease, autosomal dominant druzen, and Best's macular dystrophy.

In one approach, the compositions of the invention are administered through an ocular device suitable for direct implantation into the vitreous of the eye. The compositions of the invention may be provided in sustained release compositions, such as those described in, for example, U.S. Pat. Nos. 5,672,659 and 5,595,760. Such devices are found to provide sustained controlled release of various compositions to treat the eye without risk of detrimental local and systemic side effects. An object of the present ocular method of delivery is to maximize the amount of drug contained in an intraocular device or implant while minimizing its size in order to prolong the duration of the implant. See, e.g., U.S. Patents 5,378,475; 6,375,972, and 6,756,058 and U.S. Publications 20050096290 and 200501269448. Such implants may be biodegradable and/or biocompatible implants, or may be non-biodegradable implants. Biodegradable ocular implants are described, for example, in U.S. Patent Publication No. 20050048099. The implants may be permeable or impermeable to the active agent, and may be inserted into a chamber of the eye, such as the anterior or posterior chambers or may be implanted in the schlera, transchoroidal space, or an avascularized region exterior to the vitreous. Alternatively, a contact lens that acts as a depot for compositions of the invention may also be used for drug delivery. I

In a preferred embodiment, the implant may be positioned over an avascular region, such as on the sclera, so as to allow for transcleral diffusion of the drug to the desired site of treatment, e.g. the intraocular space and macula of the eye. Furthermore, the site of transcleral diffusion is preferably in proximity to the macula. Examples of implants for delivery of an a composition include, but are not limited to, the devices described in U.S. Pat. Nos. 3,416,530; 3,828,777; 4,014,335; 4,300,557; 4,327,725; 4,853,224; 4,946,450; 4,997,652; 5,147,647; 5,164,188; 5,178,635; 5,300,114; 5,322,691; 5,403,901; 5,443,505; 5,466,466; 5,476,511; 5,516,522; 5,632,984; 5,679,666; 5,710,165; 5,725,493; 5,743,274; 5,766,242; 5,766,619; 5,770,592; 5,773,019; 5,824,072; 5,824,073; 5,830,173; 5,836,935; 5,869,079, 5,902,598; 5,904,144; 5,916,584; 6,001,386; 6,074,661; 6,110,485; 6,126,687; 6,146,366; 6,251,090; and 6,299,895, and in WO 01/30323 and WO 01/28474, all of which are incorporated herein by reference.

Examples include, but are not limited to the following: a sustained release drug delivery system comprising an inner reservoir comprising an effective amount of an agent effective in obtaining a desired local or systemic physiological or pharmacological effect, an inner tube impermeable to the passage of the agent, the inner tube having first and second ends and covering at least a portion of the inner reservoir, the inner tube sized and formed of a material so that the inner tube is capable of supporting its own weight, an impermeable member positioned at the inner tube first end, the impermeable member preventing passage of the agent out of the reservoir through the inner tube first end, and a permeable member positioned at the inner tube second end, the permeable member allowing diffusion of the agent out of the reservoir through the inner tube second end; a method for administering a compound of the invention to a segment of an eye, the method comprising the step of implanting a sustained release device to deliver the compound of the invention to the vitreous of the eye or an implantable, sustained release device for administering a compound of the invention to a segment of an eye; a sustained release drug delivery device comprising: a) a drug core comprising a therapeutically effective amount of at least one first agent effective in obtaining a diagnostic effect or effective in obtaining a desired local or systemic physiological or pharmacological effect; b) at least one unitary cup essentially impermeable to the passage of the agent that surrounds and defines an internal compartment to accept the drug core, the unitary cup comprising an open top end with at least one recessed groove around at least some portion of the open top end of the unitary cup; c) a permeable plug which is permeable to the passage of the agent, the permeable plug is positioned at the open top end of the unitary cup wherein the groove interacts with the permeable plug holding it in position and closing the open top end, the permeable plug allowing passage of the agent out of the drug core, through the permeable plug, and out the open top end of the unitary cup; and d) at least one second agent effective in obtaining a diagnostic effect or effective in obtaining a desired local or systemic physiological or pharmacological effect; or a sustained release drug delivery device comprising: an inner core comprising an effective amount of an agent having a desired solubility and a polymer coating layer, the polymer layer being permeable to the agent, wherein the polymer coating layer completely covers the inner core.

Other approaches for ocular delivery include the use of liposomes to target a compound of the present invention to the eye, and preferably to retinal pigment epithelial cells and/or Bruch's membrane. For example, the compound may be complexed with liposomes in the manner described above, and this compound/liposome complex injected into patients with an ocular PCD, using intravenous injection to direct the compound to the desired ocular tissue or cell. Directly injecting the liposome complex into the proximity of the retinal pigment epithelial cells or Bruch's membrane can also provide for targeting of the complex with some forms of ocular PCD. In a specific embodiment, the compound is administered via intra-ocular sustained delivery (such as VITRASERT or ENVISION). In a specific embodiment, the compound is delivered by posterior subtenons injection. In another specific embodiment, microemulsion particles containing the compositions of the invention are delivered to ocular tissue to take up lipid from Bruch's membrane, retinal pigment epithelial cells cells, or both.

Nanoparticles are a colloidal carrier system that has been shown to improve the efficacy of the encapsulated drug by prolonging the serum half-life. Polyalkylcyanoacrylates (PACAs) nanoparticles are a polymer colloidal drug delivery system that is in clinical development, as described by Stella et al., J. Pharm. Sci., 2000. 89: p.1452-1464; Brigger et al., Int. J. Pharm., 2001. 214: p. 37-42; Calvo et al., Pharm. Res., 2001. 18: p. 1157-1166; and Li et al., Biol. Pharm. Bull., 2001. 24: p. 662-665. Biodegradable poly (hydroxyl acids), such as the copolymers of poly (lactic acid) (PLA) and poly (lactic-co-glycolide) (PLGA) are being extensively used in biomedical applications and have received FDA approval for certain clinical applications. In addition, PEG-PLGA nanoparticles have many desirable carrier features including (i) that the agent to be encapsulated comprises a reasonably high weight fraction (loading) of the total carrier system; (ii) that the amount of agent used in the first step of the encapsulation process is incorporated into the final carrier (entrapment efficiency) at a reasonably high level; (iii) that the carrier have the ability to be freeze-dried and reconstituted in solution without aggregation; (iv) that the carrier be biodegradable; (v) that the carrier system be of small size; and (vi) that the carrier enhance the particles persistence.

Nanoparticles are synthesized using virtually any biodegradable shell known in the art. In one embodiment, a polymer, such as poly (lactic-acid) (PLA) or poly (lactic-co-glycolic acid) (PLGA) is used. Such polymers are biocompatible and biodegradable, and are subject to modifications that desirably increase the photochemical efficacy and circulation lifetime of the nanoparticle. In one embodiment, the polymer is modified with a terminal carboxylic acid group (COOH) that increases the negative charge of the particle and thus limits the interaction with negatively charge nucleic acid aptamers. Nanoparticles are also modified with polyethylene glycol (PEG), which also increases the half-life and stability of the particles in circulation. Alternatively, the COOH group is converted to an N-hydroxysuccinimide (NHS) ester for covalent conjugation to amine-modified aptamers.

Biocompatible polymers useful in the composition and methods of the invention include, but are not limited to, polyamides, polycarbonates, polyalkylenes, polyalkylene glycols, polyalkylene oxides, polyalkylene terepthalates, polyvinyl alcohols, polyvinyl ethers, polyvinyl esters, polyvinyl halides, polyvinylpyrrolidone, polyglycolides, polysiloxanes, polyurethanes and copolymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetage phthalate, carboxylethyl cellulose, cellulose triacetate, cellulose sulphate sodium salt, poly(methyl methacrylate), poly(ethylmethacrylate), poly(butylmethacrylate), poly(isobutylmethacryla- te), poly(hexlmethacrylate), poly(isodecylmethacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene poly(ethylene glycol), poly(ethylene oxide), poly(ethylene terephthalate), poly(vinyl alcohols), poly(vinyl acetate, poly vinyl chloride polystyrene, polyvinylpryrrolidone, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutylmethacrylate), poly(hexlmethacrylate), poly(isodecl methacrylate), poly(lauryl methacrylate), poly(phenyl methacrylate), poly(methyl acrylate), poly(isopropyl acrylate), poly(isobutyl acrylate), poly(octadecl acrylate) and combinations of any of these. In one embodiment, the nanoparticles of the invention include PEG-PLGA polymers.

Compositions of the invention may also be delivered topically. For topical delivery, the compositions are provided in any pharmaceutically acceptable excipient that is approved for ocular delivery. Preferably, the composition is delivered in drop form to the surface of the eye. For some application, the delivery of the composition relies on the diffusion of the compounds through the cornea to the interior of the eye.

Those of skill in the art will recognize that the best treatment regimens for using compounds of the present invention to treat an ocular PCD can be straightforwardly determined. This is not a question of experimentation, but rather one of optimization, which is routinely conducted in the medical arts. *In vivo* studies in nude mice often provide a starting point from which to begin to optimize the dosage and delivery regimes. The frequency of injection will initially be once a week, as has been done in some mice studies. However, this frequency might be optimally adjusted from one day to every two weeks to monthly, depending upon the results obtained from the initial clinical trials and the needs of a particular patient.

Human dosage amounts can initially be determined by extrapolating from the amount of compound used in mice, as a skilled artisan recognizes it is routine in the art to modify the dosage for humans compared to animal models. In certain embodiments it is envisioned that the dosage may vary from between about 1 mg compound/Kg body weight to about 5000 mg compound/Kg body weight; or from about 5 mg/Kg body weight to about 4000 mg/Kg body weight or from about 10 mg/Kg body weight to about 3000 mg/Kg body weight; or from about 50 mg/Kg body weight to about 2000 mg/Kg body weight; or from about 100 mg/Kg body weight to about 1000 mg/Kg body weight; or from about 150 mg/Kg body weight to about 500 mg/Kg body weight. In other embodiments this dose may be about 1, 5, 10, 25, 50, 75, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1600, 1700, 1800, 1900, 2000, 2500, 3000, 3500, 4000, 4500, 5000 mg/Kg body weight. In other embodiments, it is envisaged that higher does may be used, such doses may be in the range of about 5 mg compound/Kg body to about 20 mg compound/Kg body. In other embodiments the doses may be about 8, 10, 12, 14, 16 or 18 mg/Kg body weight. Of course, this dosage amount may be adjusted upward or downward, as is routinely done in such treatment protocols, depending on the results of the initial clinical trials and the needs of a particular patient.

### Screening Assays

As discussed herein, misfolded proteins often interfere with the normal biological function of cells and cause PCD. In many cases, the accumulation of misfolded proteins in protein aggregates causes cellular damage and cytotoxicity. Useful compounds correct or prevent protein misfolding by increasing the amount of a mutant protein that is in a biochemically active conformation. Any number of methods are available for carrying out screening assays to identify such compounds. In one approach, a mutant protein that fails to adopt a wild-type protein conformation is expressed in a cell (e.g., a cell *in vitro* or *in vivo*); the cell is contacted with a candidate compound; and the effect of the compound on the conformation of the mutant protein is assayed using any method known in the art or described herein. A compound that increases the yield of correctly folded protein present in the contacted cell relative to a control cell that was not contacted with the compound, is considered useful in the methods of the invention. An increase in the amount of a correctly folded protein is assayed, for example, by measuring the protein's absorption at a characteristic wavelength (e.g., 500 nm for rhodopsin), by measuring a decrease in intracellular protein aggregation by measuring a decrease in cytotoxicity, by measuring the mitigation of a PCD-related phenotype, or by measuring an increase in the biological activity of the protein using any standard method (e.g., enzymatic activity, association with a ligand). In a related approach, the screen is carried out in the presence of 11-cis-retinal, 9-cis-retinal, or an analog or derivative thereof. Useful compounds increase the amount of protein in a biochemically functional conformation by at least 10%, 15%, or 20%, or preferably by 25%, 50%, or 75%; or most preferably by at least 100%, 200%, 300% or even 400%.

If desired, the efficacy of the identified compound is assayed in an animal model having a PCD (e.g., an animal model of retinitis pigmentosa, cystic fibrosis, Huntington's disease, Parkinsons disease, Alzheimer's disease, nephrogenic diabetes insipidus, cancer (e.g., cancer related to p53 mutations), and prion-related disorders (e.g., Jacob-Creutzfeld disease)).

### Test Compounds and Extracts

In general, compounds capable of increasing the amount of a correctly folded protein in a cell are identified from large libraries of either natural product or synthetic (or semisynthetic) extracts or chemical libraries according to methods known in the art. Those skilled in the field of drug discovery and development will understand that the precise source of test extracts or compounds is not critical to the screening procedure(s) of the invention. Accordingly, virtually any number of chemical extracts or compounds can be screened using the methods described herein. Examples of such extracts or compounds include, but are not limited to, plant-, fungal-, prokaryotic- or animal-based extracts, fermentation broths, and synthetic compounds, as well as modification of existing compounds. Numerous methods are also available for generating random or directed synthesis (e.g., semi-synthesis or total synthesis) of any number of chemical compounds, including, but not limited to, saccharide-, lipid-, peptide-, and nucleic acid-based compounds. Synthetic compound libraries are commercially available from Brandon Associates (Merrimack, N.H.) and Aldrich Chemical (Milwaukee, Wis.). Alternatively, libraries of natural compounds in the form of bacterial, fungal, plant, and animal extracts are commercially available from a number of sources, including Biotics (Sussex, UK), Xenova (Slough, UK), Harbor Branch Oceangraphics Institute (Ft. Pierce, Fla.), and PharmaMar, U.S.A. (Cambridge, Mass.). In addition, natural and synthetically produced libraries are produced, if desired, according to methods known in the art, e.g., by standard extraction and fractionation methods. Furthermore, if desired, any library or compound is readily modified using standard chemical, physical, or biochemical methods.

In addition, those skilled in the art of drug discovery and development readily understand that methods for dereplication (e.g., taxonomic dereplication, biological dereplication, and chemical dereplication, or any combination thereof) or the elimination of replicates or repeats of materials already known for their activity in correcting a misfolded protein should be employed whenever possible.

When a crude extract is found to correct the conformation of a misfolded protein further fractionation of the positive lead extract is necessary to isolate chemical constituents responsible for the observed effect. Thus, the goal of the extraction, fractionation, and purification process is the careful characterization and identification of a chemical entity within the crude extract that increase the yield of a correctly folded protein. Methods of fractionation and purification of such heterogenous extracts are known in the art. If desired, compounds shown to be useful agents for the treatment of any pathology related to a misfolded protein or protein aggreagation are chemically modified according to methods known in the art.

### Combination Therapies

Compositions of the invention useful for the treatment of a PCD (e.g., retinitis pigmentosa, Huntington's disease, Parkinson's disease, Alzheimer's disease, nephrogenic diabetes insipidus, cancer, and prion-related disorders, such as Jacob-Creutzfeld disease) may, if desired, be administered in combination with any standard therapy known in the art. For retinitis pigmentosa, standard therapies include vitamin A supplements. In the case of Parkinson's disease, standard therapies include the administration of any one or more of the following dopamine receptor agonists levodopa/carbidopa, amantadine, bromocriptine, pergolide, apomorphine, benserazide, lysuride, mesulergine, lisuride, lergotrile, memantine, metergoline, piribedil, tyramine, tyrosine, phenylalanine, bromocriptine mesylate, pergolide mesylate; other standard therapies include antihistamines, antidepressants, dopamine agonists, monoamine oxidase inhibitors. For Huntington's disease, standard therapies include the administration of any one or more of the following haloperidol, phenothiazine, reserpine, tetrabenazine, amantadine, and co-Enzyme Q10. For Alzheimer's disease standard therapies include the administration of any one or more of the following: donepezil (Aricept), rivastigmine (Exelon), galantamine (Razadyne), and tacrine (Cognex). For nephrogenic diabetes insipidus standard therapies include the administration of any one or more of the following: chlorothiazide/hydrochlorothiazide, amiloride, and indomethacin. For cancer, standard therapies-include the administration of any one or more of the following: abiraterone acetate, altretamine, anhydrovinblastine, auristatin, bexarotene, bicalutamide, BMS184476, 2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-proly-1-Lproline-t-butylamide, cachectin, cemadotin, chlorambucil, cyclophosphamide, 3',4'-didehydro-4'-deoxy-8'-norvin-caleukoblastine, docetaxol, doxetaxel, cyclophosphamide, carboplatin, carmustine (BCNU),cisplatin, cryptophycin, cytarabine, dacarbazine (DTIC), dactinomycin, daunorubicin, dolastatin, doxorubicin (adriamycin), etoposide, 5-fluorouracil, finasteride, flutamide, hydroxyurea and hydroxyureataxanes, ifosfamide, liarozole, lonidamine, lomustine (CCNU), mechlorethamine (nitrogen mustard), melphalan, mivobulin isethionate, rhizoxin, sertenef, streptozocin, mitomycin, methotrexate, nilutamide, onapristone, paclitaxel, prednimustine, procarbazine, RPR109881, stramustine phosphate, tamoxifen, tasonermin, taxol, tretinoin, vinblastine, vincristine, vindesine sulfate, and vinflunin.

### Kits

The invention provides kits for the treatment or prevention of a PCD or symptoms thereof. In one embodiment, the kit includes a pharmaceutical pack comprising an effective amount of 11-cis-retinal or 9-cis-retinal and any one or more of the following: a proteasomal inhibitor (e.g., MG132), an autophagy inhibitor (e.g., 3-methyladenine), a lysosomal inhibitor (e.g., ammonium chloride), an inhibitor of protein transport from the ER to the Golgi (e.g., brefeldin A), an Hsp90 chaperone inhibitor (e.g., Geldanamycin), a heat shock response activator (e.g., Celastrol), a glycosidase inhibitor (e.g., castanospermine) and a histone deacetylase inhibitor (e.g., Scriptaid). Preferably, the compositions are present in unit dosage form. In some embodiments, the kit comprises a sterile container which contains a therapeutic or prophylactic composition; such containers can be boxes, ampules, bottles, vials, tubes, bags, pouches, blister-packs, or other suitable container forms known in the art. Such containers can be made of plastic, glass, laminated paper, metal foil, or other materials suitable for holding medicaments.

If desired compositions of the invention or combinations thereof are provided together with instructions for administering them to a subject having or at risk of developing a PCD. The instructions will generally include information about the use of the compounds for the treatment or prevention of a PCD. In other embodiments, the instructions include at least one of the following: description of the compound or combination of compounds; dosage schedule and administration for treatment of a PCD or symptoms thereof; precautions; warnings; indications; counter-indications; overdosage information; adverse reactions; animal pharmacology; clinical studies; and/or references. The instructions may be printed directly on the container (when present), or as a label applied to the container, or as a separate sheet, pamphlet, card, or folder supplied in or with the container.

The following examples are provided to illustrate the invention, not to limit it. Those skilled in the art will understand that the specific constructions provided below may be changed in numerous ways, consistent with the above described invention while retaining the critical properties of the compounds or combinations thereof.

### Recombinant Polypeptide Expression

Because compositions of the invention increase the recovery of recombinant polypeptides having a biochemically active conformation, they are generally useful for enhancing the expression of virtually any recombinant polypeptide known in the art. In particular, compositions of the invention are useful for enhancing the recovery of biologically active polypeptides that tend to form aggregates of inactive proteins or that form inclusion bodies. To enhance recovery of biologically active forms of such proteins, at least one or more of the compositions of the invention is added to the media of a recombinant cell (e.g., a eukaryotic cell, mammalian cell, or yeast cell) expressing the protein at the time that protein synthesis is induced. Such compositions include a proteasomal inhibitor, an autophagy inhibitor, a lysosomal inhibitor, an inhibitor of protein transport from the ER to the Golgi, an Hsp90 chaperone inhibitor, a glycosidase inhibitor, a heat shock response activator, or a histone deacetylase inhibitor. To increase the expression of a recombinant or mutant opsin, 11-cis-retinal or 9-cis-retinal may be added to the media at the time of induction.

In general, recombinant polypeptides are produced by transformation of a suitable host cell with all or part of a polypeptide-encoding nucleic acid molecule or fragment thereof in a suitable expression vehicle. Those skilled in the field of molecular biology will understand that any of a wide variety of expression systems may be used to provide the recombinant protein. The precise host cell used is not critical to the invention. A recombinant polypeptide may be produced in virtually any eukaryotic host (e.g., *Saccharomyces cerevisiae*, insect cells, e.g., Sf21 cells, or mammalian cells, e.g., NIH 3T3, HeLa, or preferably COS cells). Such cells are available from a wide range of sources (e.g., the American Type Culture Collection, Rockland, Md.; also, see, e.g., Ausubel et al., Current Protocol in Molecular Biology, New York: John Wiley and Sons, 1997). The method of transfection and the choice of expression vehicle will depend on the host system selected. Transformation methods are described, e.g., in Ausubel et al. (supra); expression vehicles may be chosen from those provided, e.g., in Cloning Vectors: A Laboratory Manual (P. H. Pouwels et al., 1985, Supp. 1987).

A variety of expression systems exist for the production of recombinant polypeptides. Expression vectors useful for producing such polypeptides include, without limitation, chromosomal, episomal, and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof.

Once the recombinant polypeptide is expressed, it is isolated, e.g., using affinity chromatography. In one example, an antibody (e.g., produced as described herein) raised against the polypeptide may be attached to a column and used to isolate the recombinant polypeptide. Lysis and fractionation of polypeptide-harboring cells prior to affinity chromatography may be performed by standard methods (see, e.g., Ausubel et al., supra). Once isolated, the recombinant protein can, if desired, be further purified, e.g., by high performance liquid chromatography (see, e.g., Fisher, Laboratory Techniques In Biochemistry and Molecular Biology, eds., Work and Burdon, Elsevier, 1980).

### EXAMPLES

Retinitis pigmentosa (RP) comprises a heterogeneous group of inherited retinal disorders that lead to rod photoreceptor death. The death of photoreceptors results in night blindness and subsequent tunnel vision due to the progressive loss of peripheral vision in patients suffering from retinitis pigmentosa. Between 20-25% of patients with Autosomal Dominant Retinitis Pigmentosa (ADRP) have a mutation in the rhodopsin gene, the most common mutation being P23H. The P23H mutation results in a misfolded opsin protein that fails to associate with 11-cis-retinal. The misfolded P23H protein is retained within cells, where it forms aggregates (Saliba et. al. 2002. JCS 115: 2907-2918; Illing et. al. 2002. JBC 277: 34150-34160). This aggregation behavior classifies some RP mutations, including P23H, as protein conformational disorders (PCD).

While the following examples are directed to the use of the P23H mutant protein for the identification of compounds that reduce misfolded protein aggregation and increase the yield of correctly folded protein, the invention is not so limited. Compounds identified as useful for increasing the yield of correctly folded P23H in a cell are not only useful for the treatment of retinitis pigmentosa. Such compounds are likely to increase the yield of any misfolded protein, and are generally useful for the treatment of virtually any protein conformational disorder.

Rescue of misfolded and aggregated proteins has increasingly been shown using specific pharmacological chaperones. Competitive enzyme inhibitors have also been used as pharmacological chaperones, sometimes called specific chemical chaperones in diseases including Fabry's, GM1-gangliosidosis, Gaucher, Tay-sachs, and RP 17. To determine whether inhibition of the processes generally used by cells for protein folding, transport and degradation could increase the yield of properly folded proteins in a cell, exemplary compounds of the following classes were tested: proteasomal inhibitors, autophagy inhibitors, lysosomal inhibitors, inhibitors of protein transport from the ER to the Golgi, Hsp90 chaperone inhibitors, heat shock response activators, glycosidase inhibitors, and histone deacetylase inhibitors.

Previous studies have shown that the native chromophore 11-*cis* retinal quantitatively promotes the *in vivo* folding and stabilization of P23H opsin, as does 9-*cis*- -retinal and a 7-ring locked isomer of 11-cis-retinal (Noorwez et. al. J Biol Chem. 2003 Apr 18;278(16):14442-50). Like the wild-type protein, the rescued mutant P23H protein formed pigment, acquired mature glycosylation and was transported to the cell surface. In the present study, the effect of inhibitors of various cellular enzymes and pathways that may participate in the intracellular fate of P23H opsin is analyzed.

### Example 1: A P23H opsin expressing cell line was used to assay protein folding

Mutant P23H and wild-type opsins were expressed separately in tetracycline-inducible stable HEK293 cell lines in the presence of 11-*cis* retinal and various inhibitors. At forty-eight hours, the folded proteins were immunoaffinity purified and quantitated by UV-visible spectroscopy. The total amount of opsin protein was assayed at 280 nm. The amount of rhodopsin present in a biochemically functional conformation was assayed at 500 nm. Immunofluorescence microscopy was also performed to determine the cellular location of the proteins.

### Example 2: Proteasomal inhibition increased the recovery of correctly folded P23H.

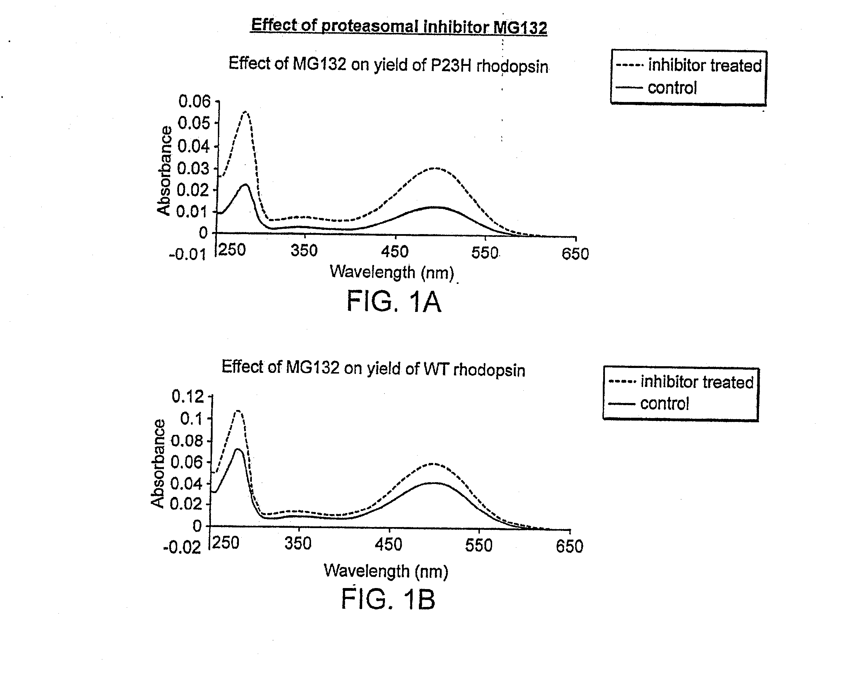

MG132, a reversible inhibitor of the proteasome, was added to the culture medium of the HEK293 cell line described in Example 1 at the time of induction. Proteasomal inhibition resulted in the recovery of more than 200-250% rhodopsin as shown in Figure 1A. In contrast, the yield of wild-type rhodopsin increased by only 35-40% (Figure 1B).

### Example 3: Autophagy inhibition increased the recovery of correctly folded P23H.

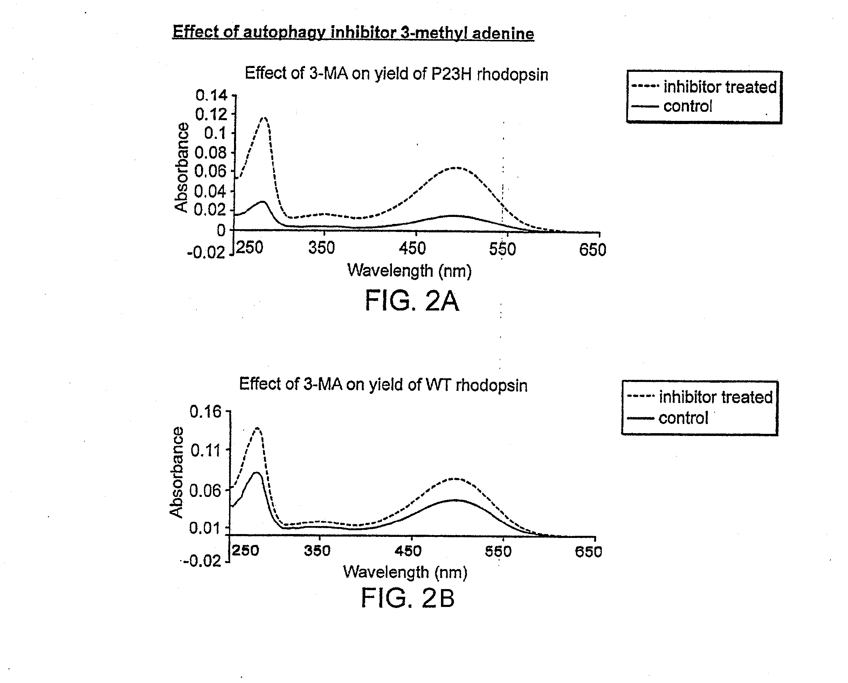

Autophagy was blocked in the HEK293 cells of Example 1 by adding 3-methyladenine to the culture medium at the time of induction. This lead to a 350-400% increase in the recovery of P23H rhodopsin while only 50-60% more wild-type rhodopsin was recovered (Figures 2A and 2B).

### Example 4: Lysosomal inhibition increased the recovery of correctly folded P23H.

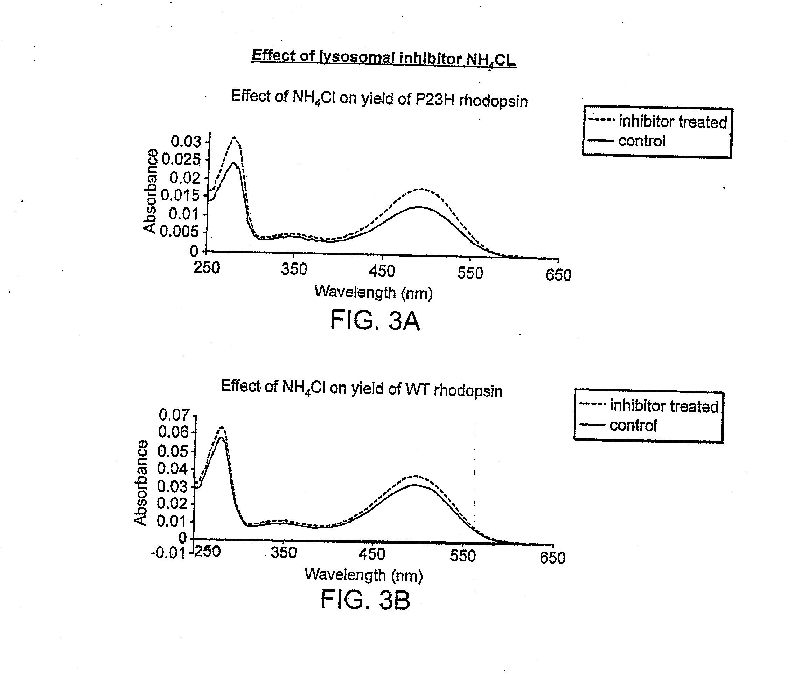

Ammonium chloride, a lysosomal inhibitor, was added to the culture medium of the HEK293 cells of Example 1 at the time that P23H protein synthesis was induced. Interestingly, lysosomal inhibition lead to a 30% increase in the recovery of P23H rhodopsin and a 10%-increases in the recovery of wild-type rhodopsin (Figures 3A and 3B).

### Example 5: Blocking ER-Golgi transport increased the yield of correctly folded P23H

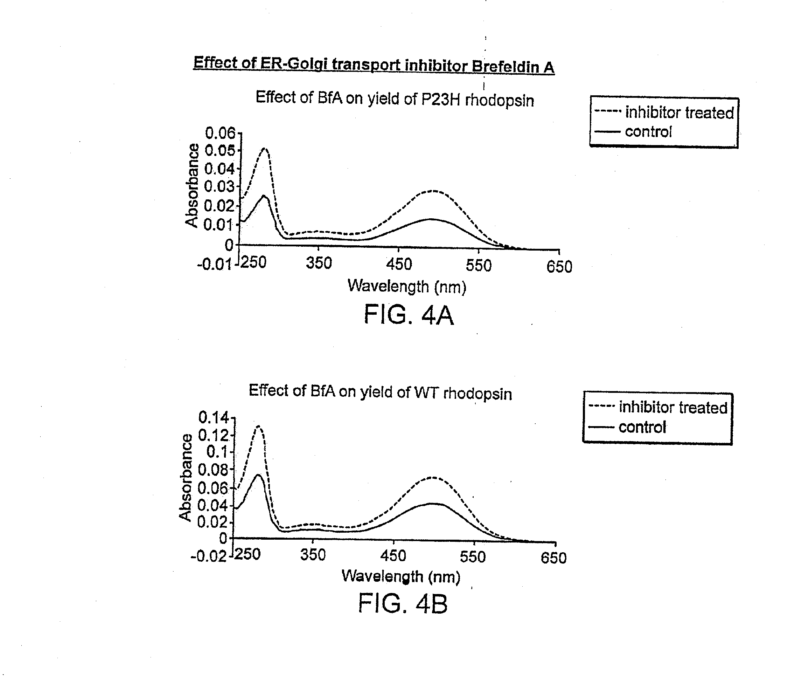

Anterograde transport of proteins from the ER to Golgi was blocked in the HEK293 cells of Example 1 by adding brefeldin A at the time of induction. This treatment resulted in a 2-fold increase in the yield of P23H rhodopsin (Figure 4A). The corresponding increase in yield for wild-type rhodopsin was about 60% (Figure 4B).

### Example 6: Hsp90 inhibition increased the recovery of correctly folded P23H

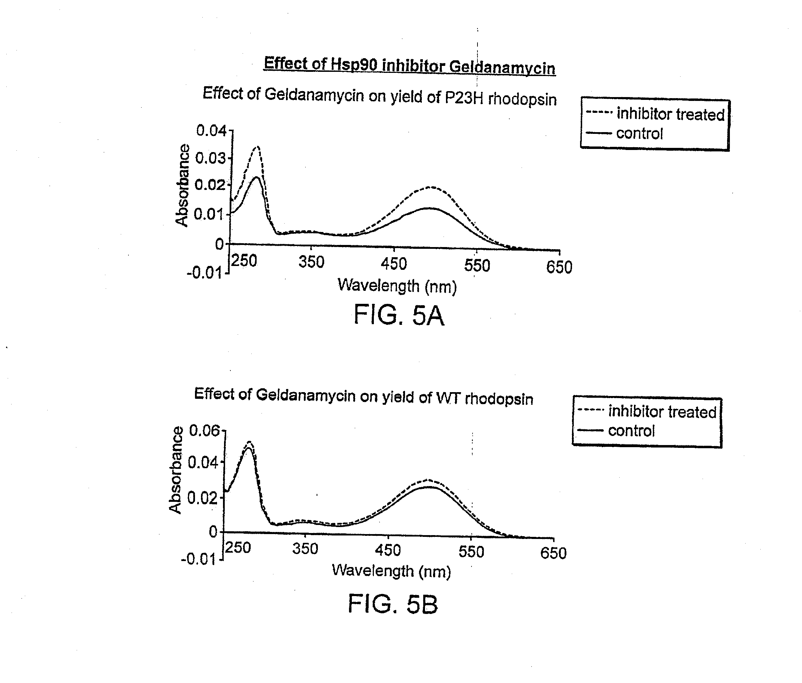

A specific Hsp90 chaperone inhibitor, Geldanamycin, was added to the culture media of the HEK293 cells described in Example 1 at the time of induction. Administration of Geldanamycin to cells expressing wild-type and P23H opsins lead to a 60% increase in the recovery of P23H rhodopsin (Figure 5A). There was only a negligible increase in the recovery of wild-type rhodopsin (Figure 5B).

### Example 7: Heat shock response activation increases the yield of correctly folded P23H

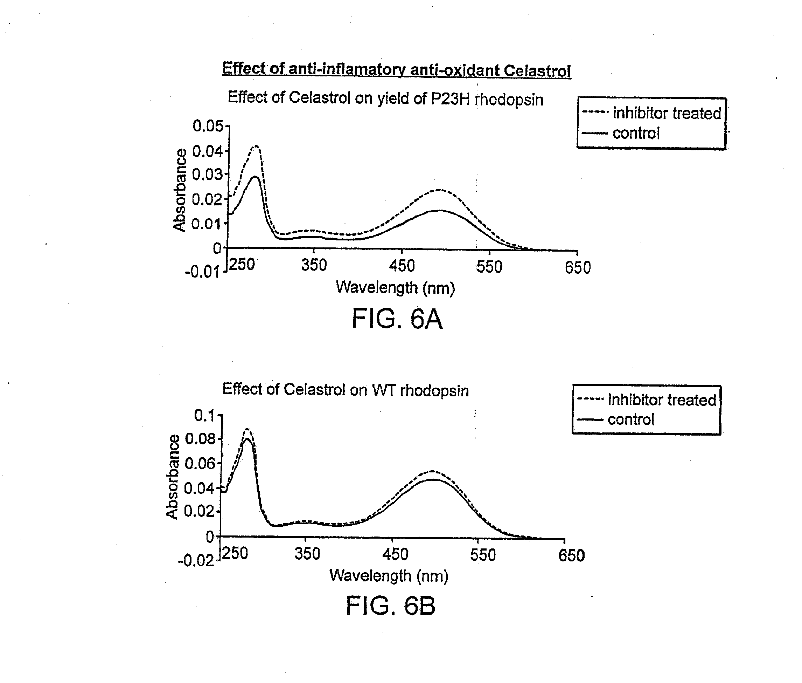

The heat shock response was activated in the HEK293 cells of Example 1 by adding Celastrol at the time of induction. This treatment resulted in a 40% increase in P23H rhodopsin (Figure 6A). Celestrol had much less of an effect on the recovery of wild-type rhodopsin (∼5-7%) (Figure 6B).

### Example 8: Dihydro-celastrol increased the recovery of wild-type and P23H rhodopsins

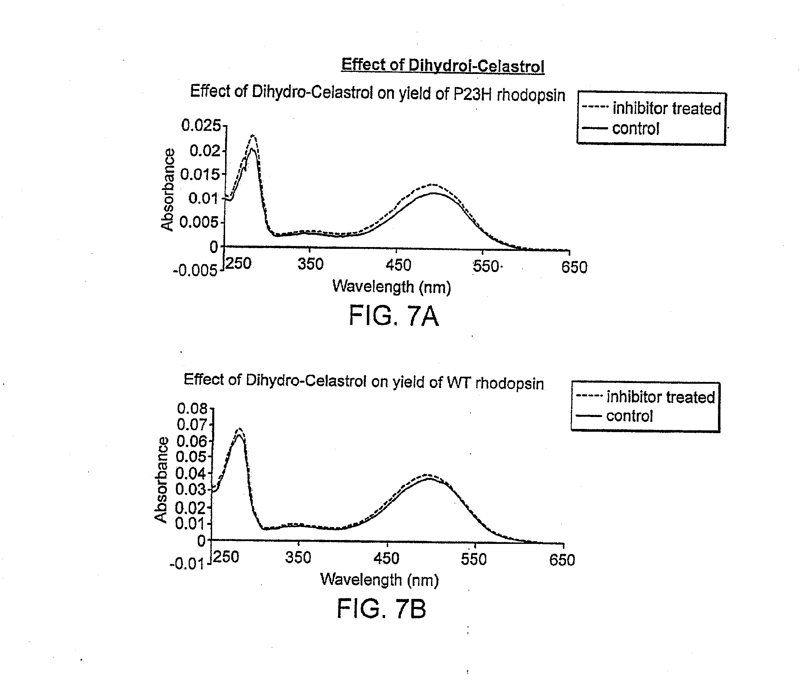

The effect of a dihydro-celastrol, a derivative of celastrol, on P23H rhodopsin recovery was assayed in the HEK293 cells of Example 1. Dihydro-celastrol increased the recovery of both wild-type and P23H rhodopsins by about 5-10% (Figures 7A and 7B).

### Example 9: Histone deacetylase inhibition increased the recovery of wild-type and P23H rhodopsins

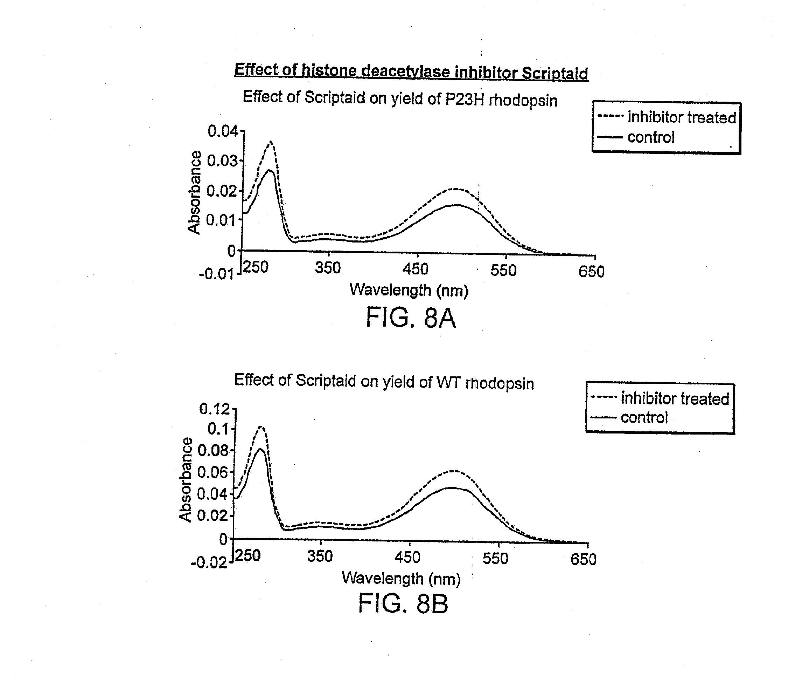

A histone deacetylase inhibitor, Scriptaid, was added to the culture media of the HEK293 cells described in Example 1 at the time of induction. Scriptaid increased the recovery of both wild-type and P23H rhodopsins by about 30% (Figures 8A and 8B).

### Example 10: 11-cis Retinal enhanced rescue of opsins in presence of inhibitors

As shown in Table 1, each of the following inhibitors increased the recovery of folded rhodopsins in the presence of 11-cis-retinal: glucosidase 1 & 2 (Castanospermine), the anterograde transport from the ER to the Golgi (Brefeldin A), Hsp90 (Geldanamycin), the proteasome (MG132), autophagy (3-MA) and lysosomes (ammonium chloride). MG132 and 3-methyladenine showed the most dramatic effect on recovery of the folded rhodopsins.

**Table 1: Effect of Inhibitors on Rhodopsin Recovery**

| **Compounds** | **P23H (% increase in recovery)** | **WT (% increase in recovery)** |
|---|---|---|
| MG132 | 230 | 140 |
| 3MA | 400 | 159 |
| Ammonium chloride | 138 | 115 |
| Brefeldin A | 210 | 160 |
| Geldanamycin | 170 | 115 |
| Celastrol | 150 | 115 |
| Dihydro celastrol acetate | 123 | 105 |
| Scriptaid | 131 | 130 |

To determine whether the effect of the inhibitors requires the presence of the specific chaperone 11-*cis*-retinal, each of the inhibitors was tested alone. MG132 which is a proteasomal inhibitor increased recovery of correctly folded P23H rhodopsin by 150% in the absence of 11-cis-retinal. 3-methyladenine, which is an autophagic inhibitor, increased recovery of correctly folded P23H rhodopsin by 200% in the absence of 11-*cis* retinal. Reduced yields of correctly folded P23H opsins were recovered when the cells were grown in the presence of the other inhibitors without the addition of 11-cis-retinal.

The P23H mutation destabilizes opsin and causes its aggregation inside the cell. Incorrectly folded P23H is incapable of binding its natural ligand, 11-cis-retinal. Addition of 11-cis-retinal to the culture medium of cells expressing the P23H mutant makes the ligand accessible to the early folding opsin intermediates and a 5-6 fold increase in rhodopsin yield is achieved. This effect is specific for the mutant and no effect is seen when 11-cis-retinal is added to the media of cells expressing wild-type opsin. The combination of 11-cis-retinal and each of a proteasomal inhibitor, an autophagy inhibitor, a lysosomal inhibitor, an inhibitor of protein transport from the ER to the Golgi, an Hsp90 chaperone inhibitor, a heat shock response activator, or a histone deacetylase inhibitor increased the recovery of P23H in a biochemically functional conformation.

A P23H protein having a biochemically functional conformation exhibits the biological activity of the wild-type protein in a functional assay. For example, a P23H protein, when expressed as described herein, is capable of being activated by light and converting to metarhodopsin II. This conversion is monitored spectrophometrically. In addition, a P23H protein, when expressed as described herein, may be isolated as part of a cell membrane. When transducin is added to the isolated membrane containing P23H, the P23H protein is capable of activating the heterotrimeric G protein transducin and triggering the exchange of GDP for GTP by the α subunit of transducin. In an animal model of retinitis pigmentosa, administration of the compositions of the invention (e.g., 11-cis-retinal in combination with any one or more of a proteasomal inhibitor, an autophagy inhibitor, a lysosomal inhibitor, an inhibitor of protein transport from the ER to the Golgi, an Hsp90 chaperone inhibitor, a heat shock response activator, a glycosidase inhibitor or a histone deacetylase inhibitor) would be expected to functionally rescue or ameliorate the symptoms associated with the retinitis pigmentosa phenotype.

### Maternal and Methods

### Cell Lines and Culture Conditions.

Stable wild-type and P23H opsin expressing HEK293 cell lines were generated in the Flp-In T-Rex system (Invitrogen). The HEK293 cells were grown in DMEM high glucose media supplemented with 10% fetal bovine serum, antibiotic-antimycotic solution, 5 µg/ml blasticidin and hygromycin at 37°C in the presence of 8% CO₂.

### Induction of Opsin Production and Addition of Inhibitors.

The opsin expressing HEK293 cell lines were allowed to reach confluence and were induced with 1 µg/ml tetracycline after a change of media. 10 µM 11-cis retinal was immediately added after induction under a red light, and inhibitors were added concurrently at the concentrations shown in Table 2 (below).

**Table 2**

| **Inhibitors used** | **Concentrations used** |
|---|---|
| MG132 | 250nM |
| 3-methyladenine | 10mM |
| Ammonium chloride | 30mM |
| Brefeldin A | 100ng/ml |
| Geldanamycin | 750nM |
| Celastrol | 1µM |
| Dihydro-celastrol | 1µM |
| Scriptaid | 4µM |
| Kifunensine | 20µM |

The plates were incubated for forty-eight hours. 10 uM of retinal was provided twenty-four hours after the first application, under red light.

### Harvesting of Cells and Rhodopsin Purification.

Forty-eight hours after induction and inhibitor administration the HEK293 cells were harvested and rhodopsin was purified essentially as described in Noorwez et. al. (J Biol Chem. 2004 Apr 16;279(16):16278-84). Spectrophotometric scans were taken in a Varian Cary 50 spectrophotometer.

### Other Embodiments

From the foregoing description, it will be apparent that variations and modifications may be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

## Claims

1. A histone deacetylase inhibitor optionally in combination with 11-cis-retinal, 9-cis-retinal or a 7-ring locked isomer of 11-cis retinal for use in treating a disease or condition selected from the group consisting of retinitis pigmentosa, age-related macular degeneration, glaucoma, corneal dystrophies, retinoschises, Stargardt's disease, autosomal dominant druzen and Best' macular dystrophy.

2. The inhibitor for the use according to claim 1 wherein the histone deacetylase inhibitor is selected from the group consisting of Scriptaid, APHA Compound 8, Apicidin, sodium butyrate, (-)-Depudecin, Sirtinol, trichostatin A, or salts thereof.

3. The inhibitor for the use according to claim 2 wherein the histone deacetylase inhibitor is Scriptaid.

4. The inhibitor for the use according to claim 2 wherein the disease or condition is retinitis pigmentosa.

5. The inhibitor for the use according to claim 2 wherein the disease or condition is age-related macular degeneration.

6. The inhibitor for the use according to claim 2 wherein the disease or condition is autosomal dominant druzen.

7. A histone deacetylase inhibitor optionally in combination with 11-cis-retinal, 9-cis-retinal or a 7-ring locked isomer of 11-cis retinal for use in treating a disease or condition selected from the group consisting of retinitis pigmentosa, age-related macular degeneration, glaucoma, corneal dystrophies, retinoschises, Stargardt's disease, autosomal dominant druzen and Best's macular dystrophy and wherein such diseases are **characterized by** the deposition of protein aggregates or fibrils within the eye.

8. A pharmaceutical composition comprising a histone deacetylase inhibitor or salts thereof in combination with a compound selected from the group consisting of 11-cis-retinal, 9-cis-retinal or a 7-ring locked isomer of 11-cis retinal and a pharmaceutically acceptable excipient.

9. The pharmaceutical composition according to claim 8 wherein the histone deacetylase inhibitor is selected from the group consisting of Scriptaid, APHA Compound 8, Apicidin, sodium butyrate, (-)-Depudecin, Sirtinol, trichostatin A, or salts thereof.

10. The pharmaceutical composition according to claim 9 wherein the histone deacetylase inhibitor is Scriptaid.
